# EUROPEAN PATENT SPECIFICATION

(11) **EP 1 440 380 B1**
(45) Date of publication and mention of the grant of the patent: **23.09.2009**
(21) Application number: 02770121.8
(22) Date of filing: 30.10.2002
(51) Int. Cl.: G06F 17/00, A61B 5/0476, A61B 5/048

(54) **IMPROVEMENTS IN OR RELATING TO PHYSIOLOGICAL MONITORING**
VERBESSERUNG BETREFFENDE PHYSIOLOGISCHE ÜBERWACHUNG
AMELIORATION D'UN MONITORAGE PHYSIOLOGIQUE

(30) Priority: 01.11.2001 GB 0126286
(43) Date of publication of application: 28.07.2004
(73) Proprietor: Oxford Biosignals Limited, Thame, Oxfordshire OX9 3EZ (GB)
(72) Inventor: POTTER, Charles, David, Ogilvy Denham House, Standlake, Oxon OX29 7SG (GB); McGROGAN, Nicholas 13 Wallingford Road, Wallingford,Oxon OX10 9LG (GB)
(74) Representative: Nicholls, Michael John
(86) International application number: PCT/GB2002/004874
(87) International publication number: WO 2003/038649

(56) References cited:
- EP-A- 0 773 504
- WO-A-00/44580
- US-A- 4 228 806
- US-A- 5 047 930
- US-A- 5 732 696
- US-A- 6 070 098
- MARMOR M F; ZRENNER E: "Standard for clinical electro-oculography. International Society for Clinical Electrophysiology of Vision." DOCUMENTA OPHTHALMOLOGICA, vol. 85, 1993, pages 115-124, XP008057702

## Description

The present invention relates to improvement in physiological monitoring, in particular sleep or vigilance monitoring.

Sleep studies currently involve the use of multiple electrodes connected to the subject to record the data necessary to make a diagnosis of a sleep disorder. A polysomnography study will typically require the following electrodes to be attached to the head, but may include many more:
- Central EEG
- Frontal EEG
- Right EOG (Electro-Oculogram)
- Left EOG
- Right EMG (Electro-Myogram)
- Left EMG
- Right and/or Left Mastoid

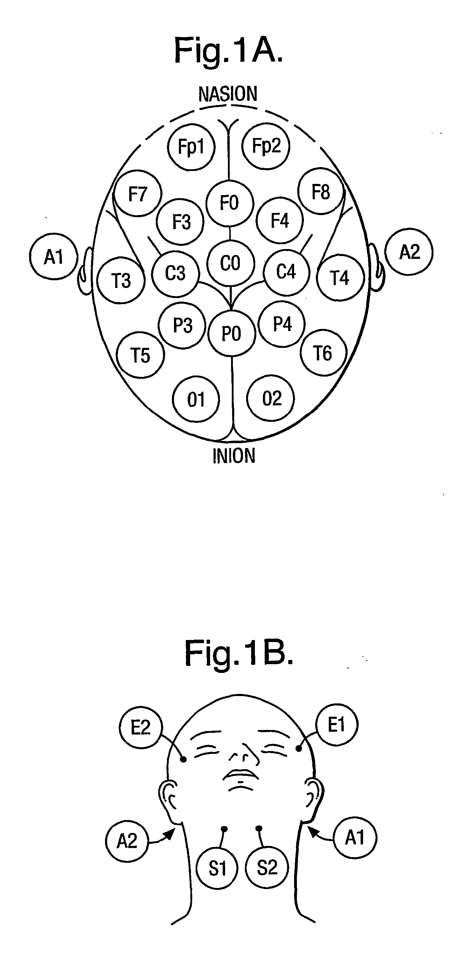

Fig. 1a and 1b of the accompanying drawings show respectively the top and view from underside of a subject's head indicating the standard positions for locating electrodes for EEG monitoring. These locations are identified in Fig. 1a and 1b by their commonly-used designations. Electrodes for electro-oculographic signals (EOG) are shown at position E1 located approximately 1 cm above and slightly lateral to the outer canthus of the left eye. Another EOG electrode is located approximately 1 cm below and slightly lateral to the outer canthus of the right eye at position E2. Positions A1 and A2 are the mastoid sites, located on the mastoid bone just behind the ear. Sites S1 and S2 are for electro-myographic signals.

Traditionally the right or left mastoid electrode has been used as a reference electrode for the others, though other "quiet" sites are possible such as the nape of the neck.

To obtain full information from the study the subject may also be required to have the following sensors attached:
- ECG electrodes (typically three)
- Thoracic respiration band
- Abdominal respiration band
- Throat microphone
- Nasal / throat airflow
- Leg sensors
- Position sensor
- Pulse oximeter

The data from the electrodes on the head is used to "score" the sleep stages of the patient throughout the recording. The method currently employed world-wide for scoring sleep recording is described in Rechtschaffen and Kales (1968), "A manual of standardized technology, techniques and scoring systems for sleep stages of human subjects". These are known as the R & K rules. Sleep scoring breaks the recording into epochs of typically 30 seconds duration and each epoch has a sleep stage classification applied to it. The six recognized classifications are: Stage wake; stage REM (rapid eye movement); stages 1, 2, 3 and 4. The classification of each epoch requires the identification of particular features in the EEG and EOG signals, and measurement of the amplitude of the EMG relative to the background EMG level. The features are identified using frequency and amplitude criteria. A set of rules is then applied to the features to obtain the classification for each epoch. Traditionally such a polysomnographic analysis is performed by a human expert visually inspecting the signal traces.

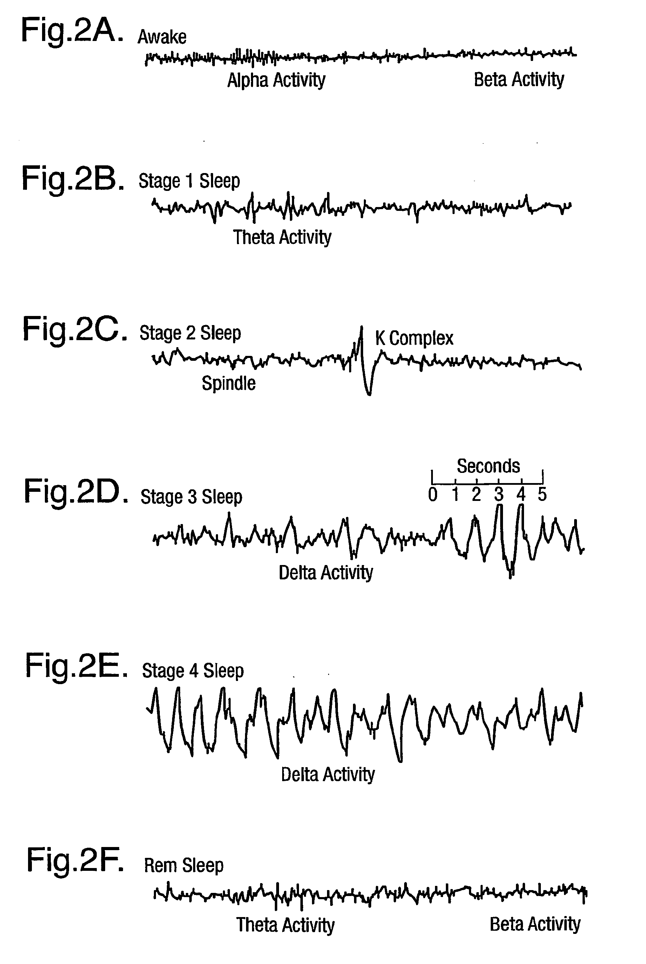

Examples of conventional EEG traces which have been assigned to the sleep stages mentioned above are shown in Fig. 2a to 2f. Fig. 2a to 2f show the following stages:
- Fig. 2a -: awake;
- Fig. 2b -: stage 1;
- Fig. 2c -: stage 2;
- Fig. 2d -: stage 3;
- Fig. 2e -: stage 4;
- Fig. 2f -: REM.

Once each epoch has been assigned a classification, clean-up rules are applied that can reclassify certain epochs according to their context. The classifications of each epoch for the entire night's recording can be plotted against time. This is known as a "hypnogram". Summary statistics can be derived from the hypnogram that allow objective measures of the quality of sleep to be made.

Thus the traditional methods of polysomnographic analysis rely on human experts, however there have been proposals for automated analysis of sleep signals.

The article "A likelihood based computer approach to conventional scoring of sleep", Procs. An. Int. Conf. of the Engineering in Medicine and Biology Society, 1992 Oct 29 to Nov 1, Vol. 14, pp 2645 to 2646 discloses a method of scoring sleep using a computer program which divides each 30 second epoch into one second intervals and calculates the likelihood of the signal in each interval matching one of eleven pre-defined features. These likelihoods are then combined into the likelihood of each of eighteen events for the epoch. The signals are the conventional five channel signals: EEG, 2EOG, 2EMG. The R& K rules can then be applied to the events by combining their probabilities using a weighting matrix to assign a sleep stage type to the epoch.

A problem with the techniques mentioned above is that the patient needs many electrodes to be attached to the head. This is inconvenient, particularly where the analysis is being performed on a patient whose sleep is probably poor anyway. Having five or more electrodes attached to their head during sleep is likely to reduce the quality of their sleep even more. Further, similar techniques are used also in vigilance monitoring, which is analogous to sleep monitoring. In vigilance monitoring the same signals are monitored, but rather than sleep stage type, wakefulness types are assigned to each epoch. Thus, for instance, the vigilance of a person operating machinery, driving or performing some safety-critical task, can be monitored. If the wakefulness stage types indicate a level of vigilance lower than required, then an alarm may be triggered. Again, the need to use five or more electrodes attached to the subject may hinder the activity of the subject Further, movement of the subject in performing their normal activities can disturb the electrodes and cause noise in the signals which confuses the analysis.

EP-A-0773504 on which the precharacterizing portions of the independent claims are based, discloses a method and apparatus for sleep and vigilance monitoring in which a trained neural network is used to analyse the signal from the mastoid sites of a subject's head. It can generate from this signal a hypnogram or series of wakefulness stage types, and can generate from these a summary of the quality.of sleep, or trigger a vigilance alarm defending on the application. The use of only the mastoid sites is advantageous in reducing the number of electrodes which have to be attached to the subject. Analysis of the signals from the mastoid sites allow, in the case of sleep, the characterisation of each epoch into one of the following three sleep stage types: Wake, REM/light or deep. Thus it does not provide a characterization of all six sleep stage types, but nevertheless requires the use of fewer electrodes, and provides for automated real-time analysis.

According to the present invention there is provided a method of sleep monitoring in accordance with claim 1, or a method of vigilance monitoring in accordance with claim 2.

In particular the present invention provides for distinguishing between the different stages including between REM and light sleep by use of the EOG signal. The EOG signal alone is analysed, this involving use of one EOG electrode (attached to the left or right EOG site), and a reference electrode. The reference electrode may be connected to the nape of the neck, or one of the mastoid sites. It is particularly advantageous for the reference site to be the mastoid site neighbouring (ie on the same side of the head) as the EOG site. This allows a very convenient siting of electrodes.

Preferably the invention provides for the characterisation of each epoch as one of at least four types, namely wake, REM sleep, light sleep, or deep sleep, on the basis of the EOG signal alone.

In the case of sleep monitoring the process may be adapted to generate a hypnogram from the assigned sleep stage types, and to analyse the hypnogram to generate a summary index of sleep quality over the period of epochs. This summary index may be displayed on a display.

In the case of vigilance monitoring the wakefulness stage types assigned may be monitored to determine whether they meet predetermined criteria which represent a lowered level of vigilance. A message generator responsive to the wakefulness stage type monitor may generate a message, such as an alarm, when the predetermined criteria are met.

Preferably the analysis is by a trained artificial neural network.

The invention will be further described by way of example with reference to the accompanying drawings in which:-
Fig. 1a and 1b, illustrate the standard sites for positioning electrodes on the head of a patient or subject;
Fig. 2a to 2f illustrate EEG signals and assigned sleep stage types;
Fig. 3 illustrates a sleep and/or vigilance monitor apparatus in accordance with one embodiment of the present invention;
Fig. 4A and B illustrates the process of analysis in one embodiment of the present invention;
Fig. 5 and 6 show hypnograms for a full night's sleep for two different subjects, trace (1) on Fig. 5 and 6 show the expertly scored data for the full nights sleep and traces (2) and (3) on Fig. 5 and 6 show the data generated by an embodiment of the invention from the right and left EOG signals.
Fig. 7 to 10 show 30 seconds of raw data for several EEG channels for two different subjects in various sleep stage types.

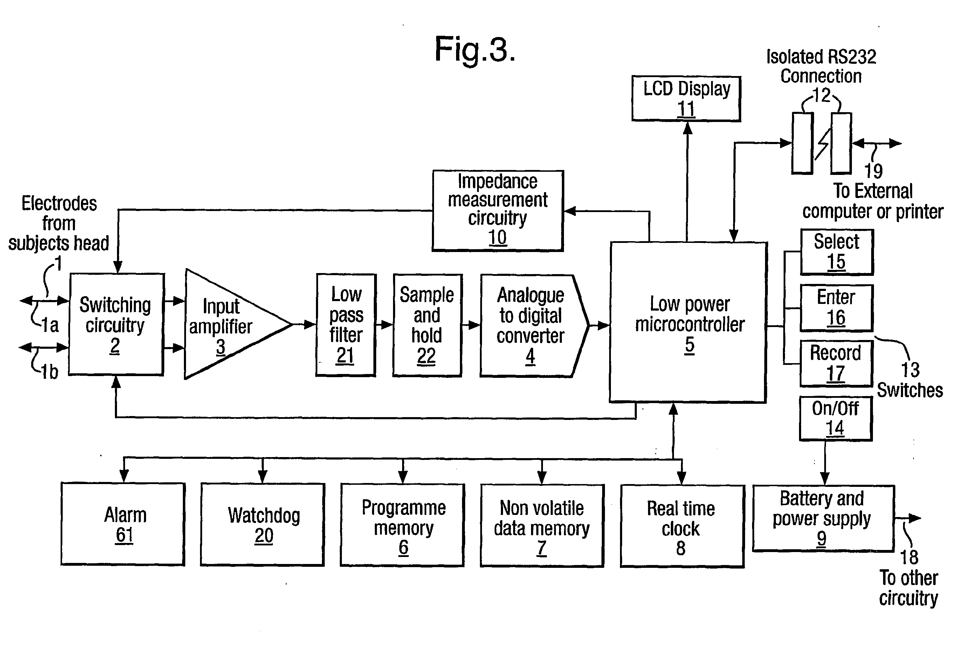

A block diagram of a typical implementation of the device according to an embodiment of the invention is described below, which refers to Fig. 3. The device is a small, self-contained portable unit which can continually acquire and analyse signals from a patient for at least 12 hours. Results are held in non-volatile data memory 7 for later display on LCD display 11 for downloading via an isolated RS232 or other link 12. The power is provided by internal primary or rechargeable batteries and a regulated power supply 9.

Signal are acquired from electrodes 1 mounted on the subject's head via switching circuitry 2 and input amplifier 3. The electrodes 1a, 1b and, where used, the indifferent electrode may be wireless electrodes thus making use of the device less obtrusive to the subject. The input amplifier has an analog bandwidth of at least 0.5 Hertz to 30 Hertz and is of a high gain, low noise instrumentation design. The signal is input to low-pass filter 21 to reduce unwanted aliasing components before analog to digital conversion using sample-and-hold circuit 22 and A/D converter 4. The resultant quantised data samples are transferred to low power microcontroller 5 for processing. In one example the sampling rate may be 128 Hertz and the quantisation of the analog to digital converter 4 be 12 bits, which provides sufficient dynamic range not to require a gain control on the input amplifier 3.

When recording one channel of EOG from the EOG site, three electrodes are typically necessary; two of them (1a, 1b) comprise the differential inputs to input amplifier 3 (one electrode being connected to an EOG site and one to a reference site such as the nearby mastoid site). The third is an "indifferent" lead (not shown) whose sole function is to allow input amplifier return currents to flow. The indifferent lead can be attached to any part of the subject's body. The indifferent lead is optional, it is possible to omit it.

Before signal acquisition begins, the impedances of the electrodes on the subject's head may be measured by causing impedance measurement circuitry 10 to drive a signal of known amplitude and source impedance via the switching circuitry 2 through the electrodes 1a, 1b in turn onto the subject's scalp. The resultant signal is measured by the microcontroller by the process described above and from it the impedances of the electrodes fitted to the subject's scalp are calculated in turn. A warning message is displayed on the LCD display 11 if the impedance of the connections to the subject's head is unacceptably high.

During data acquisition, the device continually acquires signals from the subject's head for analysis. The microcontroller 5 analyses the quantised values and from them generates results that are stored in non-volatile data memory 7. The program for the microcontroller is held in program memory 6. Real time clock 8 allows the results to be stored with a record of the time of day of acquisition.

Watchdog 20 resets the microcontroller 5 if the microcontroller fails to write to it periodically. If the microcontroller 5 is reset, it will identify whether it was in record before the reset was received and if so, go back into record so that a minimal amount of data is lost.

Alarm 61 may be activated if the device is a vigilance monitor and the processor 5 has determined that the level of vigilance of the subject is unacceptably low.

Control of the device is via switches 13, some of which can be read by the microcontroller 5. On/Off switch 14 turns the device on and off; select switch 15 displays successive prompts and results of the LCD screen 11; enter switch 16 accepts the command currently displayed on the LCD screen 11; and record switch 17 puts the device into record which starts signal acquisition, processing and storage.

When the device is switched on the user can choose whether to view the results from a previous recording; down-load results from the previous recording into a computer or to a printer; delete the results from a previous recording; or record new data.

The low-pass falter 21 can be either part of the input amplifier 3 or can be achieved in the sampling process itself within the A/D converter 4. The sleep summary index, or other results, of the analysis, can be displayed on the LCD display 11, though other types of display are possible. Further, data may be downloaded via the RS232 connection.

It will be appreciated that the above describes a specific embodiment and that variations are possible. For instance, the device may have only the means necessary to record results, with ali analysis and display earned out externally.

The signals from the electrodes are processed by microprocessor 5 using a neural network such as a multi-level perceptron (MLP). The neural network may be trained in the same manner as described in EP-A-0773504 and the assignment to each epoch of a sleep or wake stage type is also carried out in the same way as described in EP-A-0773504. In EP-A-0773504 the neural network is trained to assign sleep stage and wake stage types on the basis of a signal from the mastoid site. It is found that a neural network, trained on a central channel signal, can be used to assign sleep stage and wakefulness stage types on the EOG signal in accordance with the present invention, without retraining. However, it is also possible to train the neural network on the basis of the EOG signal directly in an analogous way to the training on the basis of the mastoid site in EP-A-0773504.

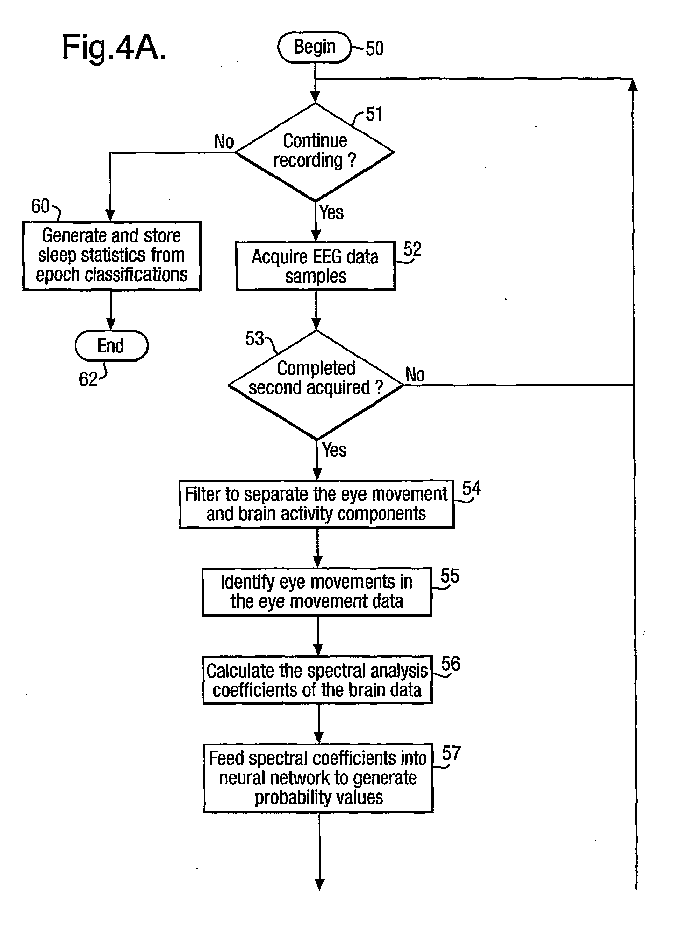

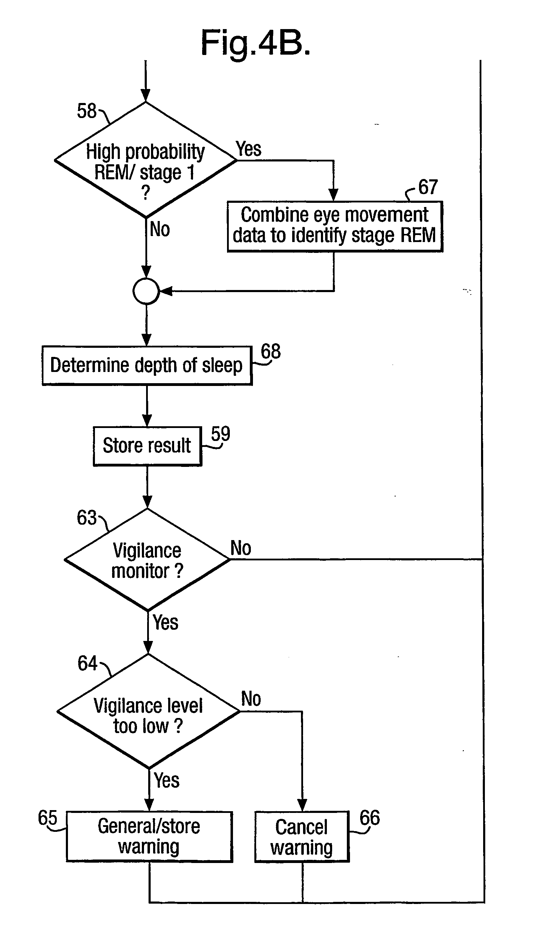

Fig. 4A and B of the accompanying drawings illustrates a flow chart for the acquisition and processing of data.

Once put into record at step 50, after checking whether the recording must continue at step 51 the microcontroller acquires data from the EOG site at step 52 until it has one second's worth of data at 53. The data is filtered at step 54 to separate the eye movement and brain activity components of the signal. Bye movements are identified within the eye movement channel at step 55. The brain activity data is processed at step 56 using a frequency domain representation to identify the dominant frequencies. There are other ways of finding the dominant frequencies. The coefficients identifying the dominant frequencies are fed at step 57 into a neural network which, in the same way as in EP-A-0773504, generates probabilities that the subject is in one of a number of sleep stages.

If the probabilities generated by the neural network indicate a high probability of sleep stage 1 or stage REM at step 58 then the eye movement data is used to distinguish between them at step 67. The distinction between stage 1 and REM sleep is possible because of the use of the EOG signal. This signal can be used to distinguish between these two types because when the subject is in REM sleep it carries low frequency eye movement structure not present during stage 1 sleep.

The probabilities of each sleep stage are combined in step 68 to form a measure of the depth of sleep. These results are then stored at step 59 and they may be displayed in any desired fashion. In the case of a vigilance monitor, the vigilance level is monitored at step 64, and if it drops below a predetermined threshold, a warning message is generated at 65. This may be used to alarm the subject, or may be stored for a later analysis.

As mentioned above, it is also possible for the microprocessor to obtain from the signals a measurement of the heart rate because there is a breakthrough into the EOG channel of an ECG signal.

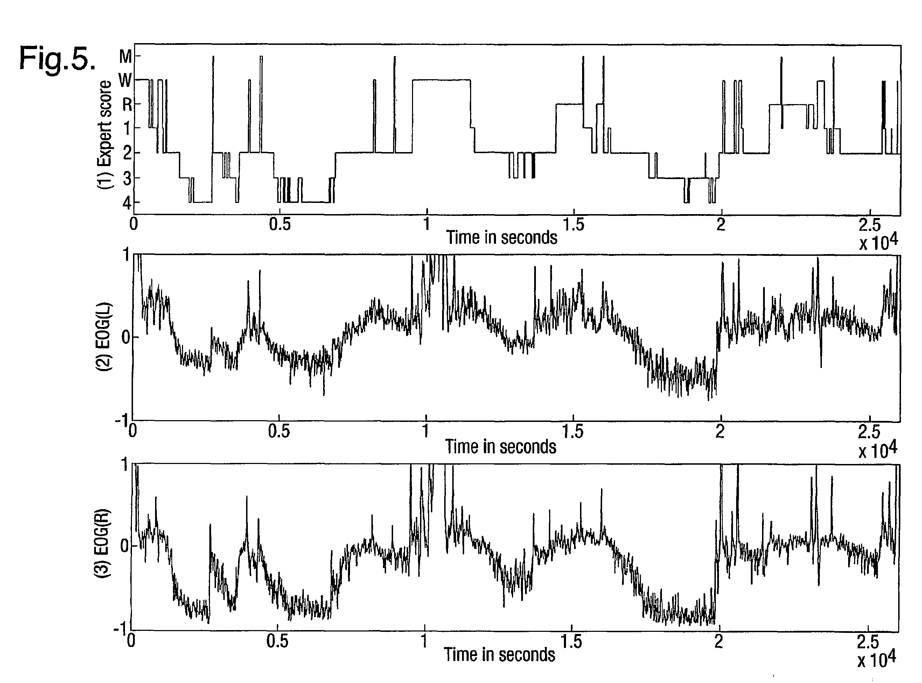

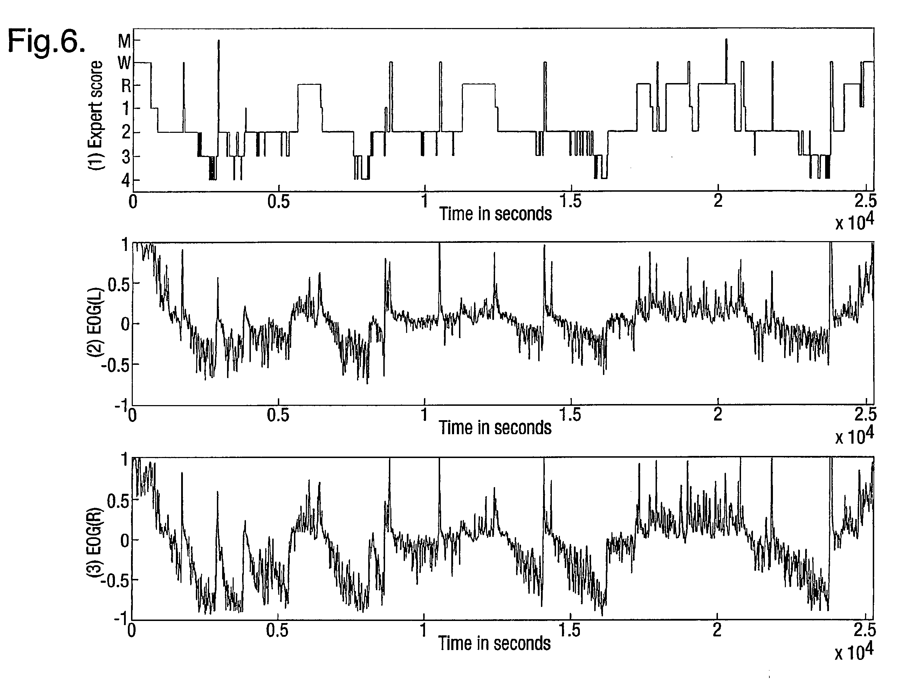

Fig. 5 to 10 show recordings from two subjects of signals from a variety of electrodes. Fig. 5 and 6 show a full nights sleep with the time in seconds along the horizontal axis. The top plot (1) in each figure shows the expert scoring in a standard R&K hypnogram in which:- M = Movement; W = Wake; and R = REM. The second and third plots show the right-side and left-side outputs EOG in a form in which the value +1 corresponds to wake, 0 corresponds to REM/light sleep and -1 corresponds to deep sleep. Although not visible in these second and third plots of Fig. 5 and 6, the present invention allows the REM to be distinguished from light sleep by displaying these two different sleep stage types in a distinguishing manner by, for example, displaying REM sleep in one colour and light sleep in another colour.

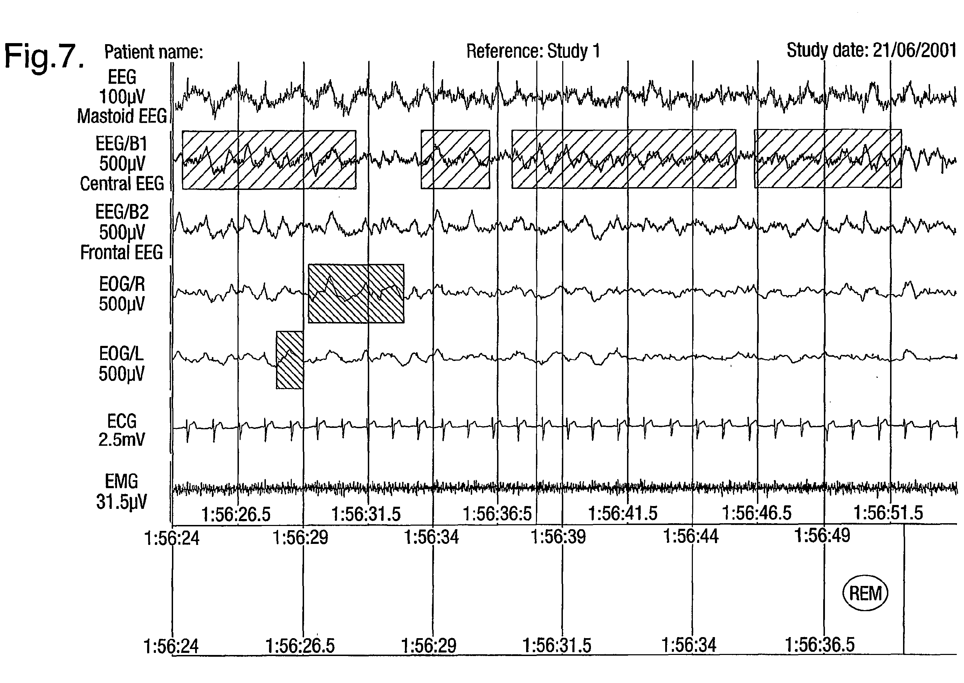

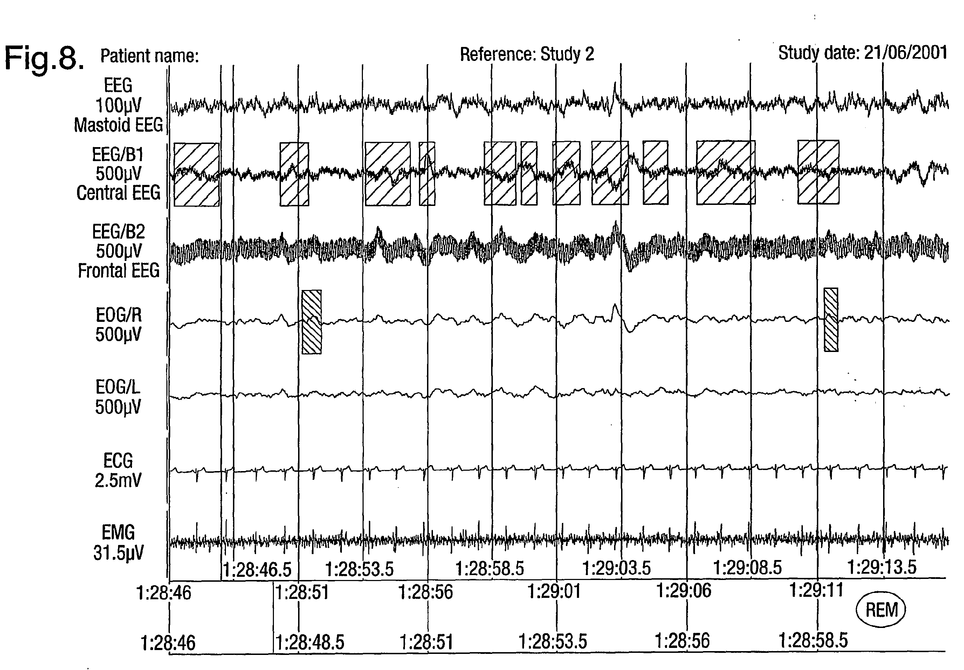

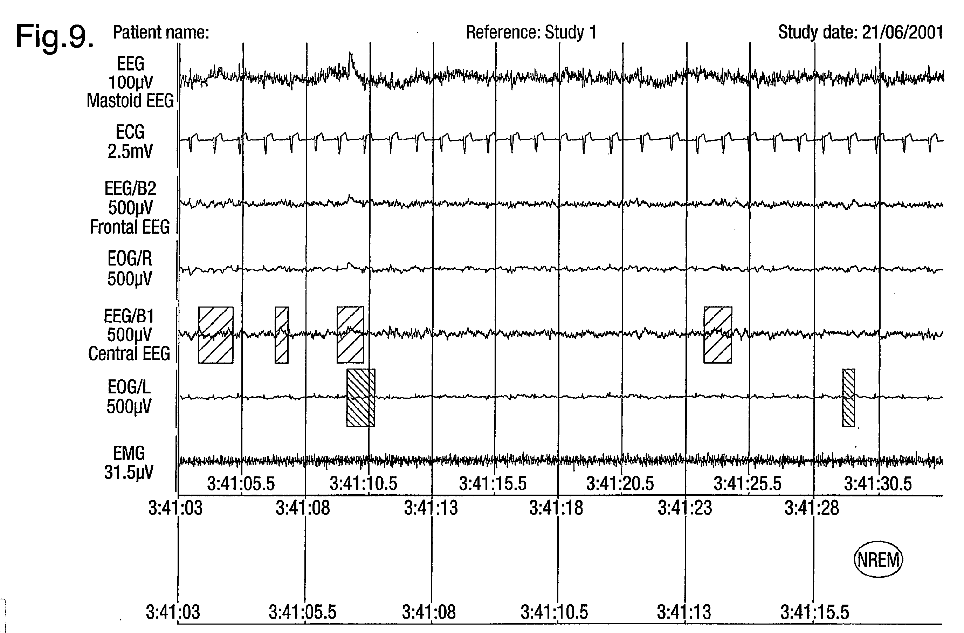

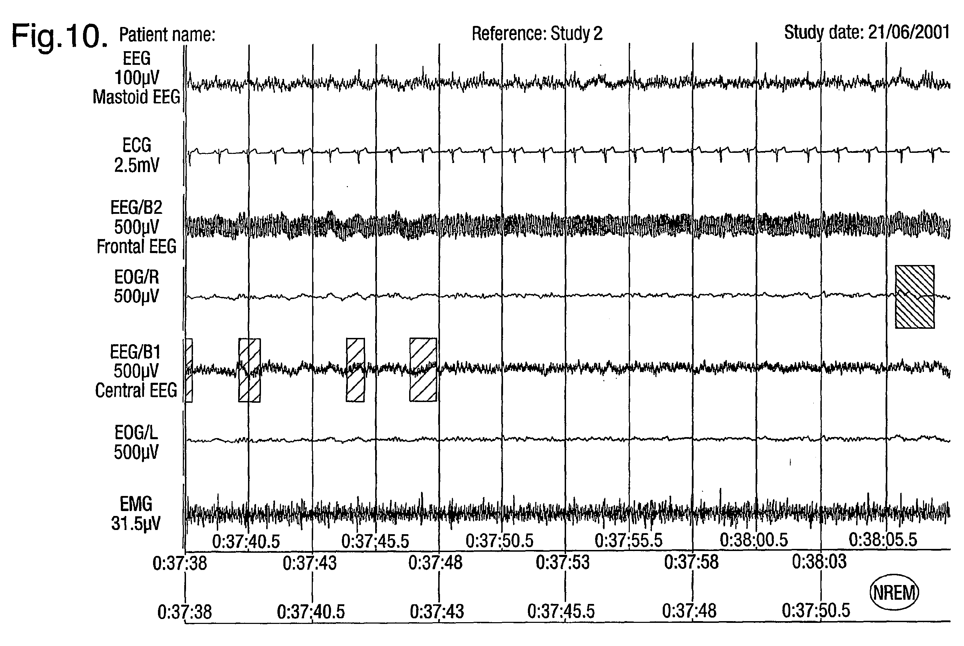

Fig. 7 to 10 show approximately 30 seconds of raw data from seven channels, including the left and right EOG channels. Fig. 7 shows a period of REM sleep for a first subject, and Fig. 8 a period of REM sleep for a second subject. In both cases the low frequency component on the EOG channels can be seen. This is particularly noticeable if compared with Fig. 9 and 10 which show 30 seconds of data for each subject respectively which correspond to a period ofnon-REM sleep. It will be seen that the low frequency eye movement structure is not present on the EOG left and right channels for either subject.

The fact that the device is compact and simple, and uses only a few electrodes, means that the accuracy of diagnosis of sleep disorders is improved because the device itself does not interfere so much with the patient's sleep. Further, it allows a first screening of patients with sleep disorders to be undertaken before the patients are referred to a specialist practitioner or sleep clinic.

The quality of sleep is also of considerable interest to elite sportsmen and women and again the ease of use of the device means that sleep monitoring is a realistic possibility for such people.

In a further embodiment the device may be used as an alarm clock which is sensitive to the quality of sleep which has been achieved and the sleep stage of the subject. Thus the device can wake the subject up after a certain number of hours of good quality sleep or alternatively wake them only during a period of light sleep or REM after a certain number of hours in bed.

As indicated above the invention is also applicable to vigilance monitoring. Vigilance research involves determining when an awake subject, normally with open eyes, starts to move from their wakeful state towards light sleep with a loss of vigilance. Many researchers have studied vigilance but no systems for alerting a loss of vigilance are in common use. The difficulty with using EEG data to score vigilance levels is that some people can operate adequately when they have significant quantities of theta activity (as shown in Figure 2B) in the EEG and might otherwise be scored as being in Stage 1 sleep. Conversely, some operators will lose vigilance while having a majority of beta activity (as shown in Figure 2A) on their EEG.

The best approach for screening the vigilance level of an operator is to take video recordings of the operator's face, and in particular their eyes, and to have this recording scored by an expert examiner. The examiner is trained to look for signs of fatigue on the recording. These signs are reflected by behavioural patterns such as "droop eyes", "slow blinks", "head nods", "eyes shut for seconds" and "yawns". The main drawback of a video system is that when the operator turns their head, the signal is lost. Some investigators have tried to circumvent this issue by mounting the video camera on the operator's head although this is not a particularly practical solution.

Using the same (preferably single) EOG channel, as above for distinguishing between REM and Light Sleep, low frequency eye movement data will be produced on the EOG signals when the eyes are moving and the eyelids are open. Signals are also detected when the eyelids open and close. The neural network is trained to distinguish between a 'fast' blink or movement of the eyes and signs of fatigue such as a slower closing of the eyes or rolling of the eyes, or long periods of no eye movement if the operator is staring into the distance, based on low frequency eye movement data on the EOG trace.

This low frequency eye movement data is used in conjunction with the standard brain wave activity on the EOG signal to produce an accurate vigilance monitor. The data may be processed by a neural network. Because of subject-to-subject differences in vigilance levels the training of the neural network may be subject specific where necessary.

## Claims

1. A method of sleep monitoring comprising: obtaining at least one signal from one electrooculogram (EOG) electrode from a subject and performing sleep analysis on the obtained signal; **characterised by** the steps of: filtering the signal (step 54) to separate the eye movement and brain activity components of the signal; identifying eye movements (step 55) within the eye movement components; processing the brain activity components (step 56) using a frequency domain representation to identify the dominant frequencies; feeding the coefficients identifying the dominant frequencies into a neural network to generate probabilities that the subject is in one of a number of sleep stages (step 57) whereby said sleep stages are assigned on the basis of the signal obtained from the EOG electrode only; and if the probabilities generated by the neural network indicate a high probability of sleep stage 1 or stage REM then the eye movement are used to distinguish between them(step 67); and combining the probabilities (step 68) to form a measure of the depth of sleep.

2. A method of vigilance monitoring comprising: performing the steps of claim 1 and further assigning (step 64) a wakefulness stage type on the basis of the signal obtained from the EOG sensor only.

3. A method according to claim 1 or 2 comprising attaching first and second electrodes (1) to respectively an EOG site and a reference site and monitoring the differential electrical signals between the two electrodes (1) to obtain said signal.

4. A method according to claim 3, wherein the reference site is the mastoid site neighbouring the EOG site.

5. A method according to any one of claims 1 to 4, wherein:
the signal is obtained from the subject over a period of epochs, the signal being related to the sleep or wakefulness stage type being experienced by the subject; and
said analysis comprises assigning a sleep or wakefulness stage type to each epoch.

6. A method according to claim 5 when dependent from claim 1, the method being for monitoring insomnia and further comprising:
analysing assigned sleep stage types to generate a summary index of sleep quality over the period of epochs; and
displaying the summary index of sleep quality.

7. A method according to claim 5 when dependent from claim 2, the method further comprising:
monitoring the assigned wakefulness stage types to determine whether they meet predetermined criteria representing a lowered level of vigilance; and
generating (step 65) an alarm message when the predetermined criteria are met.

8. A method according to any one of claims 1 to 7, wherein said analysis is performed by a trained artificial neural network.

9. Insomnia or vigilance monitoring apparatus adapted to perform the method of any one of the preceding claims and comprising:
one or more electrodes (1) to obtain said signal from said subject over a period of epochs, the signal being related to the sleep or wakefulness stage type being experienced by the subject; and
a processor (5) adapted to perform the method of anyone of the preceding claims to assign a sleep or wakefulness stage type to each epoch based on the signal obtained from the EOG sensor only.

10. An alarm clock comprising insomnia monitoring apparatus according to claim 9 and further comprising an alarm controller for triggering an alarm in accordance with predefined criteria based on the assigned sleep stage types.

11. An alarm clock in accordance with claim 10 wherein said predefined criteria comprise the condition that a predefined time has elapsed in one or more predetermined ones of the sleep stage types.

12. An alarm clock in accordance with claim 1 wherein said one or more predetermined ones of the sleep stage types include at least one of deep sleep stage types 1 to 4.

13. An alarm clock in accordance with claim 10, 11 or 12 wherein said predefined criteria comprise the condition that the currently assigned sleep stage type is a predetermined one of the sleep stage types.

14. An alarm clock in accordance with claim 13 wherein said predetermined one of the sleep stage types includes at least one of light sleep and REM sleep.

## Patentansprüche

1. Verfahren zur Schlafüberwachung, welches umfasst:
Erhalten mindestens eines Signals von einer Elektrookulogramm(EOG)-Elektrode von einer Versuchsperson und Durchführen einer Schlafanalyse anhand des erhaltenen Signals;
**gekennzeichnet durch** folgende Schritte:
Filtern des Signals (Schritt 54) zum Trennen der Augenbewegungs- und Gehirnaktivitätskomponenten des Signals; Feststellen von Augenbewegungen (Schritt 55) in den Augenbewegungskomponenten; Verarbeiten der Gehirnaktivitätskomponenten (Schritt 56) unter Verwendung einer Frequenzdomänendarstellung zum Bestimmen der dominanten Frequenzen; Eingeben der die dominanten Frequenzen bestimmenden Koeffizienten in ein neuronales Netz zum Erzeugen von Wahrscheinlichkeiten, dass die Versuchsperson sich in einer von mehreren Schlafphasen befindet (Schritt 57), wodurch die Schlafphasen auf der Grundlage des nur von der EOG-Elektrode erhaltenen Signals zugeordnet werden, und wenn die von dem neuronalen Netz erzeugten Wahrscheinlichkeiten eine hohe Wahrscheinlichkeit von Schlafphase 1 oder Schlafphase REM anzeigen, dann werden die Augenbewegungen verwendet, um zwischen diesen zu unterscheiden (Schritt 67); und Kombinieren der Wahrscheinlichkeiten (Schritt 68) zum Bilden eines Maßes der Schlaftiefe.

2. Verfahren zum Überwachen von Vigilanz, welches umfasst:
Durchführen der Schritte nach Anspruch 1 und weiterhin Zuordnen (Schritt 64) eines Wachheitsphasentyps nur auf der Grundlage des von dem EOG-Sensor erhaltenen Signals.

3. Verfahren nach Anspruch 1 oder 2, welches das Befestigen der ersten und zweiten Elektrode (1) an einer EOG-Stelle bzw. einer Bezugsstelle und das Überwachen der unterschiedlichen elektrischen Signale zwischen den zwei Elektroden (1) zum Erhalten des Signals umfasst.

4. Verfahren nach Anspruch 3, **dadurch gekennzeichnet, dass** die Bezugsstelle die benachbart zur EOG-Stelle befindliche Mastoidstelle ist.

5. Verfahren nach einem der Ansprüche 1 bis 4, **dadurch gekennzeichnet, dass:**
das Signal von der Versuchsperson über eine Dauer von Epochen erhalten wird, wobei das Signal mit dem Schlaf- oder Wachheitsphasentyp, den die Versuchsperson erfährt, in Zusammenhang steht; und
die Analyse das Zuordnen eines Schlaf- oder Wachheitsphasentyps zu jeder Epoche umfasst.

6. Verfahren nach Anspruch 5, bei Abhängigkeit von Anspruch 1, wobei das Verfahren zum Überwachen von Insomnie dient und weiterhin umfasst:
Analysieren zugeordneter Schlafphasentypen zum Erzeugen eines zusammenfassenden Index der Schlafqualität über die Dauer von Epochen; und
Anzeigen des zusammenfassenden Index der Schlafqualität.

7. Verfahren nach Anspruch 5, bei Abhängigkeit von Anspruch 2, wobei das Verfahren weiterhin umfasst:
Überwachen der zugeordneten Wachheitsphasentypen zum Ermitteln, ob sie vorbestimmte Kriterien erfüllen, die einen verringerten Grad an Vigilanz darstellen; und
Erzeugen (Schritt 65) einer akustischen Meldung, wenn die vorbestimmten Kriterien erfüllt sind.

8. Verfahren nach einem der Ansprüche 1 bis 7, **dadurch gekennzeichnet, dass** die Analyse von einem geschulten künstlichen neuronalen Netz durchgeführt wird.

9. Insomnie- oder Vigilanzüberwachungsvorrichtung, welche dafür ausgelegt ist, das Verfahren nach einem der vorstehenden Ansprüche auszuführen, und welche umfasst:
eine oder mehrere Elektroden (1), um das Signal von der Versuchsperson über eine Dauer von Epochen zu erhalten, wobei das Signal mit dem Schlaf- oder Wachheitsphasentyp in Zusammenhang steht, den die Versuchsperson erfährt; und
einen Prozessor (5), der dafür ausgelegt ist, das Verfahren nach einem der vorstehenden Ansprüche auszuführen, um jeder Epoche anhand nur des vom dem EOG-Sensor erhaltenen Signals einen Schlaf- oder Wachheitsphasentyp zuzuordnen.

10. Wecker, welcher eine Insomnieüberwachungsvorrichtung nach Anspruch 9 umfasst und welcher weiterhin ein Alarmsteuergerät zum Auslösen eines Alarms gemäß vorbestimmter Kriterien auf der Grundlage der zugeordneten Schlafphasentypen umfasst.

11. Wecker nach Anspruch 10, **dadurch gekennzeichnet, dass** die vorbestimmten Kriterien die Bedingung umfassen, dass in einem oder mehreren der Schlafphasentypen eine vorbestimmte Zeit verstrichen ist.

12. Wecker nach Anspruch 11, **dadurch gekennzeichnet, dass** eine oder mehrere der vorbestimmten Schlafphasentypen mindestens einen der Tiefschlafphasentypen von 1 bis 4 umfasst/umfassen.

13. Wecker nach Anspruch 10, 11 oder 12, **dadurch gekennzeichnet, dass** die vorbestimmten Kriterien die Bedingung umfassen, dass der aktuell zugeordnete Schlafphasentyp ein vorbestimmter der Schlafphasentypen ist.

14. Wecker nach Anspruch 13, **dadurch gekennzeichnet, dass** der vorbestimmte der Schlafphasentypen mindestens eines von leichtem Schlaf und REM-Schlaf umfasst.

## Revendications

1. Procédé de surveillance du sommeil comportant les étapes consistant à : obtenir au moins un signal depuis une électrode d'électro-oculogramme (EOG) d'un sujet et effectuer une analyse de sommeil sur le signal obtenu, **caractérisé par** les étapes consistant à : filtrer le signal (étape 54) pour séparer les composants d'activité cérébrale et de mouvement de l'oeil du signal, identifier des mouvements de l'oeil (étape 55) dans les composants de mouvement de l'oeil, traiter les composants d'activité cérébrale (étape 56) en utilisant une représentation de domaine fréquentiel pour identifier les fréquences dominantes, envoyer les coefficients identifiant les fréquences dominantes dans un réseau neuronal afin de générer des probabilités que le sujet se trouve dans une phase parmi un certain nombre de phases du sommeil (étape 57), lesdites phases de sommeil étant attribuées sur la base du signal obtenu depuis l'électrode EOG uniquement, et si les probabilités générées par le réseau neuronal indiquent une forte probabilité de phase de sommeil 1 ou de phase REM alors les mouvements de l'oeil sont utilisés pour faire la distinction entre elles (étape 67), et combiner les probabilités (étape 68) pour former une mesure de la profondeur du sommeil.

2. Procédé de surveillance de vigilance comportant : l'exécution des étapes de la revendication 1 et l'attribution en outre (étape 64) d'un type de phase de veille sur la base du signal obtenu depuis le détecteur EOG uniquement.

3. Procédé selon la revendication 1 ou 2, comportant les étapes consistant à fixer des première et seconde électrodes (1) respectivement à un site EOG et à un site de référence et à surveiller les signaux électriques différentiels entre les deux électrodes (1) pour obtenir ledit signal.

4. Procédé selon la revendication 3, dans lequel le site de référence est le site mastoïde voisin du site EOG.

5. Procédé selon l'une quelconque des revendications 1 à 4, dans lequel :
le signal est obtenu depuis le sujet sur une période, le signal étant associé au type de phase de sommeil ou de veille dans lequel le sujet se trouve, et
ladite analyse comporte l'attribution d'un type de phase de sommeil ou de veille à chaque période.

6. Procédé selon la revendication 5, lorsqu'elle est dépendante de la revendication 1, le procédé étant destiné à surveiller l'insomnie et comportant en outre les étapes consistant à :
analyser des types de phase de sommeil attribués pour générer un indice récapitulatif de qualité du sommeil sur la période, et
afficher l'indice récapitulatif de qualité du sommeil.

7. Procédé selon la revendication 5 lorsqu'elle est dépendante de la revendication 2, le procédé comporte en outre les étapes consistant à :
surveiller les types de phase de veille attribués pour déterminer si ils satisfont à des critères prédéterminés représentant un niveau réduit de vigilance, et
générer (étape 65) un message d'alarme lorsque les critères prédéterminés sont satisfaits.

8. Procédé selon l'une quelconque des revendications 1 à 7, dans lequel ladite analyse est effectuée par un réseau neuronal artificiel officiel.

9. Dispositif de surveillance d'insomnie ou de vigilance adapté pour mettre en oeuvre le procédé selon l'une quelconque des revendications précédentes et comportant :
une ou plusieurs électrodes (1) pour obtenir ledit signal depuis ledit sujet sur une période, le signal étant associé au type de phase de sommeil ou de veille dans lequel le sujet se trouve, et
un processeur (5) adapté pour mettre en oeuvre le procédé selon l'une quelconque des revendications précédentes afin d'attribuer un type de phase de sommeil ou de veille à chaque période sur la base du signal obtenu depuis le détecteur EOG uniquement.

10. Réveil comportant un dispositif de surveillance d'insomnie selon la revendication 9 et comportant en outre un dispositif de commande d'alarme pour déclencher une alarme conformément à des critères prédéfinis sur la base des types de phase de sommeil attribués.

11. Réveil selon la revendication 10, dans lequel lesdits critères prédéfinis comportent la condition qu'une durée prédéfinie soit écoulée dans un ou plusieurs types prédéterminés parmi les types de phase de sommeil.

12. Réveil selon la revendication 11, dans lequel ledit type ou lesdits types prédéterminés parmi les types de phase de sommeil inclut au moins l'un des types de phase de sommeil profond 1 à 4.

13. Réveil selon la revendication 10, 11 ou 12, dans lequel lesdits critères prédéfinis comportent la condition que le type de phase de sommeil actuellement attribué est un type prédéterminé parmi les types de phase de sommeil.

14. Réveil selon la revendication 13, dans lequel ledit type prédéterminé parmi les types de phase de sommeil inclut au moins l'un parmi un sommeil léger et un sommeil REM.
